# EUROPEAN PATENT SPECIFICATION

(11) **EP 4 181 903 B1**
(45) Date of publication and mention of the grant of the patent: **25.11.2026**
(21) Application number: 21751759.8
(22) Date of filing: 15.07.2021
(51) Int. Cl.: A61K 31/05, A61K 45/06, A61P 25/08

(54) **USE OF CANNABIDIOL IN THE TREATMENT OF SEIZURES ASSOCIATED WITH RARE EPILEPSY SYNDROMES RELATED TO GENETIC ABNORMALITIES**
VERWENDUNG VON CANNABIDIOL BEI DER BEHANDLUNG VON ANFÄLLEN IM ZUSAMMENHANG MIT SELTENEN EPILEPSIESYNDROMEN IM ZUSAMMENHANG MIT GENETISCHEN ANOMALIEN
UTILISATION DU CANNABIDIOL DANS LE TRAITEMENT DE CRISES ASSOCIÉES À DES SYNDROMES ÉPILEPTIQUES RARES LIÉS À DES ANOMALIES GÉNÉTIQUES

(30) Priority: 20.07.2020 GB 202011156
(43) Date of publication of application: 24.05.2023
(73) Proprietor: Jazz Pharmaceuticals Research UK Limited, Sittingbourne, Kent ME9 8AG (GB)
(72) Inventor: CHECKETTS, Daniel, Adam, Sittingbourne, Kent ME9 8AG (GB); CRAIG, Kevin, James, Sittingbourne, Kent ME9 8AG (GB)
(74) Representative: HGF
(86) International application number: PCT/EP2021/069904
(87) International publication number: WO 2022/017962

(56) References cited:
- WO-A1-2018/109471
- WO-A1-2019/207319
- GB-A- 2 531 282
- UNIQUE: "15q11.2 microdeletions", 2018, Retrieved from the Internet <URL:www.rarechromo.org>
- DEVIN M. COX ET AL.: "The 15q11.2 BP1-BP2 Microdeletion Syndrome: A Review", INT. J. MOL. SCI., vol. 16, 2015, pages 4068 - 4082
- GU BIN ET AL: "Cannabidiol attenuates seizures and EEG abnormalities in Angelman syndrome model mice", THE JOURNAL OF CLINICAL INVESTIGATION, vol. 129, no. 12, 2 December 2019 (2019-12-02), GB, pages 5462 - 5467, XP055832735, ISSN: 0021-9738, Retrieved from the Internet <URL:https://dm5migu4zj3pb.cloudfront.net/manuscripts/130000/130419/cache/130419.3-20191118220047-covered-e0fd13ba177f913fd3156f593ead4cfd.pdf> DOI: 10.1172/JCI130419
- CARABALLO ROBERTO ET AL: "Effectiveness of cannabidiol in a prospective cohort of children with drug-resistant epileptic encephalopathy in Argentina", SEIZURE, BAILLIERE TINDALL, LONDON, GB, vol. 80, 6 June 2020 (2020-06-06), pages 75 - 80, XP086246226, ISSN: 1059-1311, [retrieved on 20200606], DOI: 10.1016/J.SEIZURE.2020.06.005
- ROD MELONI: "University of Michigan doctor agrees to treat girl who is using medical marijuana to stop seizures", 2 April 2015 (2015-04-02), pages 1 - 4, XP055849324, Retrieved from the Internet <URL:https://www.clickondetroit.com/news/2015/04/02/university-of-michigan-doctor-agrees-to-treat-girl-who-is-using-medical-marijuana-to-stop-seizures/> [retrieved on 20211008]
- ROTARU DIANA C ET AL: "Angelman Syndrome: From Mouse Models to Therapy", NEUROSCIENCE, NEW YORK, NY, US, vol. 445, 21 February 2020 (2020-02-21), pages 172 - 189, XP086255806, ISSN: 0306-4522, [retrieved on 20200221], DOI: 10.1016/J.NEUROSCIENCE.2020.02.017
- SERENA SILVESTRO ET AL: "Use of Cannabidiol in the Treatment of Epilepsy: Efficacy and Security in Clinical Trials", MOLECULES, vol. 24, no. 8, 12 April 2019 (2019-04-12), pages 1459, XP055715278, DOI: 10.3390/molecules24081459
- EMILY STOCKINGS ET AL: "Evidence for cannabis and cannabinoids for epilepsy: a systematic review of controlled and observational evidence", JOURNAL OF NEUROLOGY NEUROSURGERY & PSYCHIATRY., vol. 89, no. 7, 6 March 2018 (2018-03-06), GB, pages 741 - 753, XP055657257, ISSN: 0022-3050, DOI: 10.1136/jnnp-2017-317168
- MORAN HAUSMAN-KEDEM ET AL: "Efficacy of CBD-enriched medical cannabis for treatment of refractory epilepsy in children and adolescents - An observational, longitudinal study", BRAIN & DEVELOPMENT, vol. 40, no. 7, 1 August 2018 (2018-08-01), NL, pages 544 - 551, XP055660691, ISSN: 0387-7604, DOI: 10.1016/j.braindev.2018.03.013
- LAUX LINDA C ET AL: "Long-term safety and efficacy of cannabidiol in children and adults with treatment resistant Lennox-Gastaut syndrome or Dravet syndrome: Expanded access program results", EPILEPSY RESEARCH, vol. 154, 25 March 2019 (2019-03-25), pages 13 - 20, XP085711365, ISSN: 0920-1211, DOI: 10.1016/J.EPLEPSYRES.2019.03.015

## Description

### FIELD OF THE INVENTION

The present invention relates to the use of cannabidiol (CBD) for the treatment of seizures associated with rare epilepsy syndromes. In particular the seizures associated with rare epilepsy syndromes that are treated are those which are experienced in patients with 15q11.2 microdeletion. In a further embodiment the types of seizures include tonic, tonic-clonic, atonic, absence, focal seizures without impairment, and focal seizures with secondary generalisation. Preferably the dose of CBD is between 5 mg/kg/day to 50 mg/kg/day.

In a further embodiment the CBD used is in the form of a highly purified extract of cannabis such that the CBD is present at greater than 95% of the total extract (w/w) and the cannabinoid tetrahydrocannabinol (THC) has been substantially removed, to a level of not more than 0.15% (w/w).

Preferably the CBD used is in the form of a botanically derived purified CBD which comprises greater than or equal to 98% (w/w) CBD and less than or equal to 2% (w/w) of other cannabinoids. More preferably the other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w). The botanically derived purified CBD preferably also comprises a mixture of both trans-THC and cis-THC. Alternatively, a synthetically produced CBD is used.

Most preferably the other cannabinoids present are THC at a concentration of about 0.01% to about 0.1% (w/w); CBD-C1 at a concentration of about 0.1% to about 0.15% (w/w); CBDV at a concentration of about 0.2% to about 0.8% (w/w); and CBD-C4 at a concentration of about 0.3% to about 0.4% (w/w). Most preferably still the THC is present at a concentration of about 0.02% to about 0.05% (w/w).

Where the CBD is given concomitantly with one or more other anti-epileptic drugs (AED), the CBD may be formulated for administration separately, sequentially or simultaneously with one or more AED or the combination may be provided in a single dosage form.

### BACKGROUND TO THE INVENTION

Epilepsy occurs in approximately 1% of the population worldwide, (Thurman *et al*., 2011) of which 70% are able to adequately control their symptoms with the available existing anti-epileptic drugs (AED). However, 30% of this patient group, (Eadie *et al*., 2012), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as "*failure of adequate trials of two tolerated and appropriately chosen and used AED schedules (whether* as *monotherapies or in combination) to achieve sustained seizure freedom*" (Kwan *et al*., 2009).

Individuals who develop epilepsy during the first few years of life are often difficult to treat and as such are often termed treatment resistant. Children who undergo frequent seizures in childhood are often left with neurological damage which can cause cognitive, behavioral and motor delays.

Childhood epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from an investigation into the type of seizures that the patient is experiencing is undertaken. Clinical observations and electroencephalography (EEG) tests are conducted and the type(s) of seizures are classified according to the ILEA classification.

Generalized seizures, where the seizure arises within and rapidly engages bilaterally distributed networks, can be split into six subtypes: tonic-clonic (grand mal) seizures; absence (petit mal) seizures; clonic seizures; tonic seizures; atonic seizures and myoclonic seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a bilateral convulsive seizure, which is the proposed terminology to replace secondary generalized seizures (generalized seizures that have evolved from focal seizures and are no longer remain localized).

Focal seizures where the subject's awareness / responsiveness is altered are referred to as focal seizures with impairment and focal seizures where the awareness or responsiveness of the subject is not impaired are referred to as focal seizures without impairment.

Gross chromosomal mutations, also known as gross chromosomal rearrangements (GCRs), are major chromosomal alterations and include translocations, inversions, large deletions, and partial or complete loss of chromosomes. Such alterations can lead to malignant transformation and are found in most cases of cancer. GCRs are thought to be triggered by DNA double strand breaks, which in turn can be spontaneous or induced by external agents (e.g. ionizing radiation).

15q11.2 microdeletion is a chromosome abnormality in which a tiny piece of genetic material on the long arm of chromosome 15 (at a location designated q11.2) is deleted. The microdeletion may occur randomly for the first time in an affected person, or it may be inherited from a parent. The features of people with a 15q11.2 microdeletion vary widely. The most common features include developmental, motor, and language delays; behavior and emotional problems; attention deficit disorders; and autism spectrum disorder. Other features may include birth defects and seizures. However, some people have no apparent physical, learning, or behavior problems. Treatment depends on the signs and symptoms in each person.

1p36 deletion syndrome (not claimed) is a chromosome disorder that typically causes severe intellectual disability. The syndrome is caused by a deletion of genetic material from a specific region in the short (p) arm of chromosome 1. Most cases are not inherited. Symptoms may include not speaking or barely speaking; temper tantrums; biting; exhibiting other behavioral problems. Most have structural abnormalities of the brain, and seizures occur in more than half of individuals with this disorder. Other features include a small head that is unusually short and wide; weak muscle tone; swallowing difficulties; vision and hearing problems; abnormalities of the skeleton, heart, gastrointestinal system, kidneys, or genitalia; and distinctive facial features. Treatment depends on the symptoms, and may include rehabilitation, antiepileptic medication, and standard treatment for heart, kidney, eye, hearing or bone problems.

22q11.2 duplication syndrome (not claimed) is a condition caused by an extra copy of a small piece of chromosome 22 which contains about 30 to 40 genes. It is typically inherited in an autosomal dominant manner but can occur as a de novo mutation. Affected individuals may have intellectual or learning disability, developmental delay, slow growth leading to short stature, and weak muscle tone. Many people with the condition have no apparent physical or intellectual disabilities. Treatment depends on the symptoms in each person and may include educational programs.

9p21.1 (not claimed) contains a gene (APTX) that encodes the protein Aprataxin. Aprataxin is a member of the histidine triad superfamily and plays a role in DNA-single-strand break repair. Mutations in APTX can cause Ataxia with oculomotor apraxia type 1 (AOA1), an autosomal-recessive neurodegenerative disorder mainly characterized by a childhood onset of slowly progressive cerebellar ataxia, oculomotor apraxia, dysarthria, limb dysmetria, motor and sensory axonal neuropathy. Clinical symptoms can also include dystonia, chorea, optic atrophy and cognitive impairment. Treatment for the condition may include speech therapy, physiotherapy and orthopaedic assessment.

Monosomy (not claimed) is a form of aneuploidy with the presence of only one chromosome from a pair and in turn partial monosomy occurs when a portion of one chromosome in a pair is missing. For example, if a female has one X chromosome (X monosomy) rather than two, she has Turner syndrome. Monosomy 16p13.3 is a type of partial monosomy whereby a small piece in a pair of chromosome 16 is missing.

Trisomy (not claimed) is a type of aneuploidy in which there are three instances of a particular chromosome, instead of the normal two. Trisomy 2p25.3 is a type of partial trisomy whereby there is an extra copy of a small piece of chromosome 2.

Chromosome 3q duplication (not claimed) is a chromosome abnormality that occurs when there is an extra copy of genetic material on the long arm (q) of chromosome 3. The severity of the condition and the signs and symptoms depend on the size and location of the duplication and which genes are involved. Features that may be present in a person with a chromosome 3q duplication include distinctive facial features, excessive hair growth in women, small head size, intellectual disability, slowed growth, and abnormalities of the hands, feet, genitourinary system, kidneys, and/or heart. About one third of babies with chromosome 3q duplication do not survive past the first year of life, often due to heart defects or infections. Chromosome 3q duplication can be de novo or inherited from a parent. Treatment is based on the signs and symptoms present in each person.

Trisomy 13 (not claimed) is a type of chromosome disorder characterized by having 3 copies of chromosome 13 in cells of the body, instead of the usual 2 copies. In some affected people, only a portion of cells contains the extra chromosome 13, whereas other cells contain the normal chromosome pair. Trisomy 13 causes severe intellectual disability and many physical abnormalities, such as congenital heart defects; brain or spinal cord abnormalities; very small or poorly developed eyes; extra fingers or toes; cleft lip with or without cleft palate; and weak muscle tone. Most cases are not inherited and result from a random error during the formation of eggs or sperm in healthy parents. Due to various life-threatening medical problems, many infants with trisomy 13 do not survive past the first days or weeks of life. Treatment for trisomy 13 depends on the affected person's signs and symptoms and is generally symptomatic and supportive.

Cannabidiol (CBD), a non-psychoactive derivative from the cannabis plant, has demonstrated anti-convulsant properties in several anecdotal reports, pre-clinical and clinical studies both in animal models and humans. Three randomized control trials showed efficacy of the purified pharmaceutical formulation of CBD in patients with Dravet and Lennox-Gastaut syndrome.

Based on these three trials, a botanically derived purified CBD preparation was approved by FDA in June 2018 for the treatment of seizures associated with Dravet and Lennox-Gastaut syndromes.

Documents such as WO 2018/109471 and Gu *et al.* (2019)¹ disclose the use of CBD to treat Angelman Syndrome, a condition caused by a mutation in chromosome 15 (15q11-13 duplication). WO 2018/057484 and Ho and Wassman (2017)² disclose the use of CBD to treat Wolf-Hirschhorn syndrome, a condition caused by mutation in chromosome 4. However, none provide any data of patients with gross chromosomal mutations as discussed above nor is there any mention of these conditions.

The applicant has found by way of an open label, expanded-access program that treatment with CBD resulted in a significant reduction in tonic, tonic-clonic, atonic, absence, focal seizures without impairment, and focal seizures with secondary generalisation in patients with gross chromosomal mutations, including 15q11.2 deletion; 1p36 deletion; 22Q11 duplication; 9p21.1 deletion with autistic spectrum disorder; monosomy 16p13.3 and trisomy 2p25.3; chromosome 3q duplication; and trisomy 13.

### BRIEF SUMMARY OF THE DISCLOSURE

In accordance with a first aspect of the present invention there is provided a cannabidiol (CBD) preparation for use in the treatment of seizures associated with gross chromosomal mutation.

The gross chromosomal mutation 15q11.2 microdeletion.

In a further embodiment, the seizures associated with the gross chromosomal mutations are tonic, tonic-clonic, atonic, absence, focal seizures without impairment, and focal seizures with secondary generalisation.

In a further embodiment, the CBD preparation comprises greater than 95% (w/w) CBD and not more than 0.15% (w/w) tetrahydrocannabinol (THC).

Preferably the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

Preferably the CBD preparation is used in combination with one or more concomitant anti-epileptic drugs (AED).

Preferably the one or more AED is selected from the group consisting of: valproic acid, levetiracetam, clobazam, zonisamide, rufinamide, lacosamide, topiramate, clonazepam, perampanel, brivaracetam, gabapentin and phenobarbital.

In one embodiment the CBD is present is isolated from cannabis plant material. Preferably at least a portion of at least one of the cannabinoids present in the CBD preparation is isolated from cannabis plant material.

In a further embodiment the CBD is present as a synthetic preparation. Preferably at least a portion of at least one of the cannabinoids present in the CBD preparation is prepared synthetically.

Preferably the dose of CBD is greater than 5 mg/kg/day. More preferably the dose of CBD is 20 mg/kg/day. More preferably the dose of CBD is 25 mg/kg/day. More preferably the dose of CBD is 50 mg/kg/day.

### DEFINITIONS

Definitions of some of the terms used to describe the invention are detailed below:

Over 100 different cannabinoids have been identified, see for example, Handbook of Cannabis, Roger Pertwee, Chapter 1, pages 3 to 15. These cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Tonic seizures" can be generalised onset, affecting both sides of the brain, or they can be focal onset, starting in just one side of the brain. If a tonic seizure starts in both sides of the brain, all muscles tighten and the subject's body goes stiff. If standing, they may fall to the floor, their neck may extend, eyes open wide and roll upwards, whilst their arms may raise upwards and legs stretch or contract. If a tonic seizure starts in one side of the brain muscles tighten in just one area of the body. Tonic seizures usually last less than one minute.

"Tonic-clonic seizures" consist of two phases: the tonic phase and the clonic phase. In the tonic phase the body becomes entire rigid, and in the clonic phase there is uncontrolled jerking. Tonic-clonic seizures may or may not be preceded by an aura, and are often followed by headache, confusion, and sleep. They may last mere seconds or continue for several minutes. These seizures are also known as a grand mal seizure.

"Atonic seizures" occur when a person suddenly loses muscle tone so their head or body may go limp. They are also known as drop attacks. In some children, only their head drops suddenly. They can begin in one area or side of the brain (focal onset) or both sides of the brain (generalized onset).

"Absence seizures" also may be called "petit mal" seizures. These types of seizure cause a loss of awareness for a short time. They mainly affect children although can happen at any age. During an absence seizure, a person may: stare blankly into space; look like they're "daydreaming"; flutter their eyes; make slight jerking movements of their body or limbs. The seizures usually only last up to 15 seconds and may occur several times a day.

"Focal Seizures" are defined as seizures which originate within networks limited to only one hemisphere. What happens during the seizure depends on where in the brain the seizure happens and what that part of the brain normally does.

"Focal seizures without impairment" are seizures which originate within networks limited to only one hemisphere where the awareness or responsiveness of the subject is not impaired.

"Focal seizure with impairment" usually start in a small area of the temporal lobe or frontal lobe of the brain and involve other areas of the brain within the same hemisphere that affect alertness and awareness. Most subjects experience automatisms during a focal seizure with impaired consciousness.

"Focal seizure with secondary generalisation" start in a limited area on one side of the brain and spread to involve both sides. This is different from a generalized onset seizure, which starts on both sides of the brain.

### DETAILED DESCRIPTION

### PREPARATION OF HIGHLY PURIFIED CBD EXTRACT

The following describes the production of the highly-purified (>95% w/w) cannabidiol extract which has a known and constant composition.

In summary the drug substance used is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD. Although the CBD is highly purified because it is produced from a cannabis plant rather than synthetically there is a small number of other cannabinoids which are co-produced and co-extracted with the CBD. Details of these cannabinoids and the quantities in which they are present in the medication are as described in Table A below.

**Table A: Composition of highly purified CBD extract**

| **Cannabinoid** | **Concentration** |
|---|---|
| CBD | > 95% w/w |
| CBDA | NMT 0.15% w/w |
| CBDV | NMT 1.0% w/w |
| Δ⁹ THC | NMT 0.15% w/w |
| CBD-C4 | NMT 0.5% w/w |

| | |
|---|---|
| > - greater than NMT - not more than | |

### PREPARATION OF BOTANICALLY DERIVED PURIFIED CBD

The following describes the production of the botanically derived purified CBD which comprises greater than or equal to 98% w/w CBD and less than or equal to other cannabinoids was used in the open label, expanded-access program described in Example 1 below.

In summary the drug substance used in the trials is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 95% CBD w/w, typically greater than 98% w/w.

The *Cannabis sativa* L. plants are grown, harvested, and processed to produce a botanical extract (intermediate) and then purified by crystallization to yield the CBD (botanically derived purified CBD).

The plant starting material is referred to as Botanical Raw Material (BRM); the botanical extract is the intermediate; and the active pharmaceutical ingredient (API) is CBD, the drug substance.

All parts of the process are controlled by specifications. The botanical raw material specification is described in Table B and the CBD API is described in Table C.

**Table B: CBD botanical raw material specification**

| **Test** | **Method** | **Specification** |
|---|---|---|
| Identification: | | |
| -A | Visual | Complies |
| -B | TLC | Corresponds to standard (for CBD & CBDA) |
| -C | HPLC/UV | Positive for CBDA |
| Assay: CBDA + CBD | In-house (HPLC/UV) | NLT 90% of assayed cannabinoids by peak area |
| Loss on Drying | Ph. Eur. | NMT 15% |
| Aflatoxin | UKAS method | NMT 4ppb |
| Microbial: | Ph.Eur. | NMT10⁷cfu/g |
| - TVC | | NMT10⁵cfu/g |
| - Fungi | | |
| - E.coli | | NMT10²cfu/g |
| Foreign Matter: | Ph.Eur. | NMT 2% |
| Residual Herbicides and Pesticides | Ph.Eur. | Complies |

**Table C: Specification of an exemplary botanically derived purified CBD preparation**

| **Test** | **Test Method** | **Limits** |
|---|---|---|
| Appearance | Visual | Off-white / pale yellow crystals |
| Identification A | HPLC-UV | Retention time of major peak corresponds to certified CBD Reference Standard |
| Identification B | GC-FID/MS | Retention time and mass spectrum of major peak corresponds to certified CBD Reference Standard |
| Identification C | FT-IR | Conforms to reference spectrum for certified CBD Reference Standard |
| Identification D | Melting Point | 65 - 67°C |
| Identification E | Specific Optical Rotation | Conforms with certified CBD Reference Standard; -110° to -140° (in 95% ethanol) |
| Total Purity | Calculation | ≥ 98.0% |
| Chromatographic Purity 1 | HPLC-UV | > 98.0% |
| Chromatographic Purity 2 | GC-FID/MS | ≥ 98.0 % |
| CBDA | HPLC-UV | NMT 0.15% w/w |
| CBDV | | 0.2-1.0% w/w |
| THC | | 0.01-0.1% w/w |
| CBD-C4 | | 0.3-0.5% w/w |
| Residual Solvents: | GC | |
| Alkane | | NMT 0.5% w/w |
| Ethanol | | NMT 0.5% w/w |
| Residual Water | Karl Fischer | NMT 1.0% w/w |

The purity of the botanically derived purified CBD preparation was greater than or equal to 98%. The botanically derived purified CBD includes THC and other cannabinoids, e.g., CBDA, CBDV, CBD-C1, and CBD-C4.

In some embodiments, the CBD preparation comprises not more than 0.15% THC based on total amount of cannabinoid in the preparation. In some embodiments, the CBD preparation comprises about 0.01% to about 0.1% THC based on total amount of cannabinoid in the preparation. In some embodiments, the CBD preparation comprises about 0.02% to about 0.05% THC based on total amount of cannabinoid in the preparation.

In some embodiments, the CBD preparation comprises about 0.2% to about 1.0% CBDV based on total amount of cannabinoid in the preparation. In some embodiments, the CBD preparation comprises about 0.2% to about 0.8% CBDV based on total amount of cannabinoid in the preparation.

In some embodiments, the CBD preparation comprises about 0.3% to about 0.5% CBD-C4 based on total amount of cannabinoid in the preparation. In some embodiments, the CBD preparation comprises about 0.3% to about 0.4% CBD-C4 based on total amount of cannabinoid in the preparation.

In some embodiments, the CBD preparation comprises about 0.1% to about 0.15% CBD-C1 based on total amount of cannabinoid in the preparation.

Distinct chemotypes of the *Cannabis sativa* L. plant have been produced to maximize the output of the specific chemical constituents, the cannabinoids. Certain chemovars produce predominantly CBD. Only the (-)-trans isomer of CBD is believed to occur naturally. During purification, the stereochemistry of CBD is not affected.

### Production of CBD botanical drug substance

An overview of the steps to produce a botanical extract, the intermediate, are as follows:
a) Growing
b) Direct drying
c) Decarboxylation
d) Extraction - using liquid CO₂
e) Winterization using ethanol
f) Filtration
g) Evaporation

High CBD chemovars were grown, harvested, dried, baled and stored in a dry room until required. The botanical raw material (BRM) was finely chopped using an Apex mill fitted with a 1 mm screen. The milled BRM was stored in a freezer prior to extraction.

Decarboxylation of CBDA to CBD was carried out using heat. BRM was decarboxylated at 115°C for 60 minutes.

Extraction was performed using liquid CO₂ to produce botanical drug substance (BDS), which was then crystalized to produce the test material. The crude CBD BDS was winterized to refine the extract under standard conditions (2 volumes of ethanol at -20°C for approximately 50 hours). The precipitated waxes were removed by filtration and the solvent was removed to yield the BDS.

### Production of botanically derived purified CBD preparation

The manufacturing steps to produce the botanically derived purified CBD preparation from BDS were as follows:
a) Crystallization using C₅-C₁₂ straight chain or branched alkane
b) Filtration
c) Vacuum drying

The BDS produced using the methodology above was dispersed in C₅-C₁₂ straight chain or branched alkane. The mixture was manually agitated to break up any lumps and the sealed container then placed in a freezer for approximately 48 hours. The crystals were isolated via vacuum filtration, washed with aliquots of cold C₅-C₁₂ straight chain or branched alkane, and dried under a vacuum of <10mb at a temperature of 60°C until dry. The botanically derived purified CBD preparation was stored in a freezer at -20°C in a pharmaceutical grade stainless steel container, with FDA food grade approved silicone seal and clamps.

### Physicochemical properties of the botanically derived purified CBD

The botanically derived purified CBD used in the clinical trial described in the invention comprises greater than or equal to 98% (w/w) CBD and less than or equal to 2% (w/w) of other cannabinoids. The other cannabinoids present are THC at a concentration of less than or equal to 0.1% (w/w); CBD-C1 at a concentration of less than or equal to 0.15% (w/w); CBDV at a concentration of less than or equal to 0.8% (w/w); and CBD-C4 at a concentration of less than or equal to 0.4% (w/w).

The botanically derived purified CBD used additionally comprises a mixture of both trans-THC and cis-THC. It was found that the ratio of the trans-THC to cis-THC is altered and can be controlled by the processing and purification process, ranging from 3.3:1 (trans-THC:cis-THC) in its unrefined decarboxylated state to 0.8:1 (trans-THC:cis-THC) when highly purified.

Furthermore, the cis-THC found in botanically derived purified CBD is present as a mixture of both the (+)-cis-THC and the (-)-cis-THC isoforms.

Clearly a CBD preparation could be produced synthetically by producing a composition with duplicate components.

Example 1 below describes the use of a botanically derived purified CBD in an open label, expanded-access program to investigate the clinical efficacy and safety of purified pharmaceutical cannabidiol formulation (CBD) in the treatment of seizures associated with gross chromosomal mutations including 15q11.2 deletion; 1p36 deletion; 22Q11 duplication; 9p21.1 deletion with autistic spectrum disorder; monosomy 16p13.3 and trisomy 2p25.3; chromosome 3q duplication; and trisomy 13.

### EXAMPLE 1: CLINICAL EFFICACY AND SAFETY OF PURIFIED PHARMACEUTICAL CANNABIDIOL (CBD) IN THE TREATMENT OF PATIENTS WITH GROSS CHROMOSOMAL MUTATIONS

### Study design

Subjects were required to be on one or more AEDs at stable doses for a minimum of two weeks prior to baseline and to have stable vagus nerve stimulation (VNS) settings and ketogenic diet ratios for a minimum of four weeks prior to baseline.

Patients were administered botanically derived purified CBD in a 100 mg/mL sesame oilbased solution at an initial dose of between 5 and 15 milligrams per kilogram per day (mg/kg/day) in two divided doses. Dose was then increased weekly by 5mg/kg/day to a goal of 20 to 25 mg/kg/day.

A maximum dose of 50 mg/kg/day could be utilised for patients who were tolerating the medication but had not achieved seizure control; these patients had further weekly titration by 5mg/kg/day.

There were seven patients in this study, and each received CBD for various durations of time. Modifications were made to concomitant AEDs as per clinical indication.

Seizure frequency, intensity, and duration were recorded by caregivers in a diary during a baseline period of at least 28 days. Changes in seizure frequency relative to baseline were calculated after at least 2 weeks and at defined timepoints of treatment.

### Statistical Methods:

Patients may be defined as responders if they had more than 50% reduction in seizure frequency compared to baseline. The percent change in seizure frequency was calculated as follows: $\begin{matrix}% change = \\ \begin{matrix}seizure \\ frequency\end{matrix}\end{matrix} \frac{\left(\left(weekly seizure frequency time interval\right)-\left(weekly seizure frequency Baseline\right)\right)}{\left(weekly seizure frequency Baseline\right)} \times 100$

The percent change of seizure frequency may be calculated for any time interval where seizure number has been recorded. For the purpose of this example the percent change of seizure frequency for the end of the treatment period was calculated as follows: $\begin{matrix}% reduction = \\ seizure frequency\end{matrix} \frac{\left(\left(weekly seizure frequency Baseline\right)-\left(weekly seizure frequency End\right)\right)}{\left(weekly seizure frequency Baseline\right)} \times 100$

### Results

### Patient description

The seven patients enrolled in the open label, expanded-access program had gross chromosomal mutations including 15q11.2 deletion; 1p36 deletion; 22Q11 duplication; 9p21.1 deletion with autistic spectrum disorder; monosomy 16p13.3 and trisomy 2p25.3; chromosome 3q duplication; and trisomy 13. These patients experienced several different seizure types including tonic, tonic-clonic, atonic, absence, focal seizures without impairment, focal seizures with impairment and focal seizures with secondary generalisation, and were taking several concomitant AEDs.

The age of patients ranged from 3-16 years, four were male and three were female as detailed in Table 1 below.

**Table 1: Patient demographics, seizure type and concomitant medication**

| **Patient Number** | **Age (years)** | **Sex** | **Seizure types** | **Concomitant AEDs** |
|---|---|---|---|---|
| **1** | 8.65 | F | Tonic-clonic | CLB, LEV, ZNS |
| **2** | 3.51 | M | Atonic | CLN |
| **3** | 6.33 | F | Tonic, tonic-clonic, absence | CLB, LCS |
| **4** | 13.68 | M | Focal without impairment, focal with secondary generalisation | CLB, PMP |
| **5** | 16.31 | M | Tonic, absence, focal without impairment | CLB, VPA, BRV |
| **6** | 10.87 | F | Atonic, focal without impairment, focal with impairment | RFN, ZNS |
| **7** | 6.75 | M | Tonic, tonic-clonic, absence | CLB, GBP, PHB, TPM |

| | | | | |
|---|---|---|---|---|
| VPA = valproic acid, LEV = levetiracetam, CLB = clobazam, ZNS = zonisamide, RFN = rufinamide, LCS = lacosamide, TPM = topiramate, CLN = clonazepam, PMP = perampanel, BRV = brivaracetam, GBP = gabapentin, PHB = phenobarbital | | | | |

### Study medication and concomitant medications

All seven patients were titrated up to at least 20 mg/kg/day of CBD and two patients were titrated up to at least 25 mg/kg/day (#1, 5). The average number of concomitant AEDs at the time of starting CBD was two per patient (range: 1-4).

### Clinical changes

Tables 2A-G illustrate the seizure frequency for each patient as well as the dose of CBD given.

**Table 2A: Seizure frequency data for Patient 1**

| **Patient 1** | | |
|---|---|---|
| **Time** | **Seizure Type** | **Dose CBD (mg/kg/day)** |
| | **Tonic-clonic** | |
| **Baseline** | 102.0 | - |
| **4 weeks** | 6.0 | 5.0 |
| **8 weeks** | 14.0 | 5.0 |
| **12 weeks** | 6.0 | 10.0 |
| **24 weeks** | 3.0 | 15.0 |
| **36 weeks** | 1.3 | 20.0 |
| **48 weeks** | 1.3 | 20.0 |
| **60 weeks** | 1.0 | 20.0 |
| **72 weeks** | 1.6 | 20.0 |
| **84 weeks** | 4.6 | 20.0 |
| **96 weeks** | 6.3 | 20.0 |
| **108 weeks** | 2.1 | 20.0 |
| **120 weeks** | 3.4 | 20.0 |

Patient 1 was treated for 120 weeks and experienced a 96.7% reduction in tonic-clonic seizures over the treatment period.

**Table 2B: Seizure frequency data for Patient 2**

| **Patient 2** | | |
|---|---|---|
| **Time** | **Seizure Type** | **Dose CBD (mg/kg/day)** |
| | **Atonic** | |
| **Baseline** | 156.0 | - |
| **2 weeks** | 120.0 | 5.0 |
| **4 weeks** | 120.0 | 10.0 |
| **8 weeks** | 120.0 | 15.0 |
| **12 weeks** | 180.0 | 25.0 |
| **24 weeks** | 104.0 | 22.5 |
| **36 weeks** | 160.0 | 25.0 |
| **48 weeks** | 320.0 | 25.0 |
| **60 weeks** | 180.0 | 25.0 |
| **72 weeks** | 20.0 | 25.0 |
| **84 weeks** | 72.0 | 25.0 |
| **96 weeks** | 148.0 | 25.0 |
| **108 weeks** | 60.0 | 25.0 |
| **120 weeks** | 88.0 | 25.0 |

Patient 2 was treated for 120 weeks and experienced a 43.6% reduction in atonic seizures over the treatment period.

**Table 2C: Seizure frequency data for Patient 3**

| **Patient 3** | | | | |
|---|---|---|---|---|
| **Time** | **Seizure Type** | | | **Dose CBD (mg/kg/day)** |
| | **Tonic** | **Tonic-clonic** | **Absence** | |
| **Baseline** | 100.0 | 8.0 | 15.0 | - |
| **4 weeks** | 20.0 | 3.0 | 1.0 | 5.0 |
| **8 weeks** | 11.0 | 2.0 | 1.0 | 10.0 |
| **12 weeks** | 28.0 | 4.0 | 0.0 | 12.5 |
| **24 weeks** | 11.3 | 0.0 | 0.3 | 15.0 |
| **36 weeks** | 16.4 | 0.0 | 0.3 | 20.0 |
| **48 weeks** | 12.0 | 0.0 | 0.6 | 25.0 |
| **60 weeks** | 5.0 | 4.3 | 0.3 | 25.0 |
| **72 weeks** | 4.0 | 0.0 | 0.0 | 25.0 |
| **84 weeks** | 5.3 | 1.3 | 0.0 | 25.0 |
| **96 weeks** | 3.7 | 0.0 | 1.8 | 25.0 |
| **108 weeks** | 0.0 | 0.0 | 0.0 | 25.0 |
| **132 weeks** | 0.0 | 0.9 | 2.4 | 25.0 |

Patient 3 was treated for 132 weeks and experienced a 100% reduction in tonic seizures, a 88.8% reduction in tonic-clonic seizures and a 84% reduction in absence seizures over the treatment period.

**Table 2D: Seizure frequency data for Patient 4**

| **Patient 4** | | | |
|---|---|---|---|
| **Time** | **Seizure Type** | | **Dose CBD (mg/kg/day)** |
| | **Focal without impairment** | **Focal with secondary generalisation** | |
| **Baseline** | 16.0 | 13.0 | - |
| **2 weeks** | 0.0 | 0.0 | 15.0 |
| **4 weeks** | 0.0 | 0.0 | 20.0 |
| **8 weeks** | 18.0 | 0.0 | 25.0 |
| **12 weeks** | 0.0 | 0.0 | 25.0 |
| **16 weeks** | 1.0 | 0.0 | 25.0 |
| **24 weeks** | 2.0 | 0.8 | 25.0 |

Patient 4 was treated for 24 weeks and experienced a 83.3% reduction in focal seizures without impairment and a 93.8% reduction in focal seizures with secondary generalisation over the treatment period.

**Table 2E: Seizure frequency data for Patient 5**

| **Patient 5** | | | | |
|---|---|---|---|---|
| **Time** | **Seizure Type** | | | **Dose CBD (mg/kg/day)** |
| | **Tonic** | **Absence** | **Focal without impairment** | |
| **Baseline** | 14.0 | 55.0 | 12.0 | - |
| **2 weeks** | 8.0 | 78.0 | 0.0 | 10.0 |
| **4 weeks** | 4.3 | 81.8 | 47.4 | 15.0 |
| **8 weeks** | 1.9 | 39.6 | 2.9 | 20.0 |
| **12 weeks** | 5.1 | 43.4 | 1.0 | 20.0 |
| **16 weeks** | 3.0 | 6.0 | 0.0 | 20.0 |
| **24 weeks** | 7.0 | 0.0 | 0.0 | 20.0 |

Patient 5 was treated for 24 weeks and experienced a 50% reduction in tonic seizures, a 100% reduction in absence seizures and a 100% reduction in focal seizures without impairment over the treatment period.

**Table 2F: Seizure frequency data for Patient 6**

| **Patient 6** | | | | |
|---|---|---|---|---|
| **Time** | **Seizure Type** | | | **Dose CBD (mg/kg/day)** |
| | **Atonic** | **Focal without impairment** | **Focal with impairment** | |
| **Baseline** | 2.0 | 3.0 | 2.0 | - |
| **2 weeks** | 0.0 | 0.0 | 4.0 | 10.0 |
| **4 weeks** | 0.0 | 0.0 | 2.0 | 20.0 |
| **8 weeks** | 0.0 | 0.0 | 2.0 | 25.0 |
| **12 weeks** | 0.0 | 1.0 | 3.0 | 25.0 |
| **16 weeks** | 2.0 | 5.0 | 2.0 | 25.0 |
| **24 weeks** | 3.0 | 6.0 | 5.0 | 25.0 |

Patient 6 was treated for 24 weeks and did not experience a reduction in seizures over the treatment period.

**Table 2G: Seizure frequency data for Patient 7**

| **Patient 7** | | | | |
|---|---|---|---|---|
| **Time** | **Seizure Type** | | | **Dose CBD (mg/kg/day)** |
| | **Tonic** | **Tonic-clonic** | **Absence** | |
| **Baseline** | 352.0 | 272.0 | 92.0 | - |
| **4 weeks** | 372.0 | 240.0 | 20.0 | 10.0 |
| **12 weeks** | 308.0 | 448.0 | 12.0 | 10.0 |
| **16 weeks** | 308.0 | 448.0 | 12.0 | 10.0 |
| **24 weeks** | 248.0 | 404.0 | 20.0 | 10.0 |
| **48 weeks** | 216.0 | 356.0 | 20.0 | 15.0 |
| **60 weeks** | 244.0 | 304.0 | 28.0 | 20.0 |
| **72 weeks** | 244.0 | 304.0 | 28.0 | 20.0 |
| **84 weeks** | 208.0 | 280.0 | 32.0 | 25.0 |
| **108 weeks** | 184.0 | 288.0 | 24.0 | 25.0 |
| **120 weeks** | 0.0 | 5.6 | 0.0 | 23.6 |
| **132 weeks** | 0.0 | 2.4 | 0.0 | 23.2 |
| **144 weeks** | 0.0 | 0.0 | 0.0 | 25.9 |

Patient 7 was treated for 144 weeks and experienced a 100% reduction in tonic seizures, a 100% reduction in tonic-clonic seizures and a 100% reduction in absence seizures over the treatment period.

Overall, patients reported reductions of 43.6-100% in seizures over period of treatment with CBD.

Significantly, one patient became completely seizure free after 144 weeks of treatment with CBD (patient 7), whilst one patient became seizure free in their tonic seizures after 108 weeks of treatment (patient 3) and one patient became seizure free in their absence seizures and focal seizures without impairment after 24 weeks and 16 weeks of treatment respectively (patient 5).

CBD was effective in reducing the frequency of the following seizure types: tonic, tonic-clonic, atonic, absence, focal seizures without impairment and focal seizures with secondary generalisation

### Conclusions

These data indicate that CBD was able to significantly reduce the number of seizures associated with gross chromosomal mutations including 15q11.2 deletion; 1p36 deletion; 22Q11 duplication; 9p21.1 deletion with autistic spectrum disorder; monosomy 16p13.3 and trisomy 2p25.3; chromosome 3q duplication; and trisomy 13. Clearly the treatment is of significant benefit in this difficult to treat epilepsy syndrome given the high response rate experienced in most patients.

Of interest are patients with tonic-clonic seizures (patients 1, 3 and 7), and patients with absence seizures (patients 3, 5 and 7) who obtained significant benefit.

In conclusion, this study signifies the use of CBD for treatment of seizures associated with gross chromosomal mutations. Seizure types include tonic, tonic-clonic, atonic, absence, focal seizures without impairment and focal seizures with secondary generalisation for which seizure frequency rates decreased significantly, by 44-100%.

### REFERENCES

1. Gu et al. (2019) "Cannabidiol attenuates seizures and EEG abnormalities in Angelman syndrome model mice." The Journal of Clinical Investigation, Vol 129, 2019; pp5462-5467
2. Ho and Wassman (2017) "A case for cannabidiol in Wolf-Hirschhorn syndrome seizure management." American Journal of Medical Genetics, Vol 173A, 2017; pp 324-326.

## Claims

1. A cannabidiol (CBD) preparation for use in the treatment of seizures associated with 15q11.2 microdeletion, wherein the seizures are selected from tonic, tonic-clonic, atonic, absence, focal seizures without impairment, and focal seizures with secondary generalisation.

2. A CBD preparation for use according to claim 1, wherein the CBD preparation comprises greater than 95% (w/w) CBD and not more than 0.15% (w/w) tetrahydrocannabinol (THC).

3. A CBD preparation for use according to any of the preceding claims, wherein the CBD preparation comprises greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), and wherein the THC is present as a mixture of trans-THC and cis-THC.

4. A CBD preparation for use according to any of the preceding claims, wherein the CBD preparation is used in combination with one or more concomitant anti-epileptic drugs (AED).

5. A CBD preparation for use according to claim 4, wherein the one or more AED is selected from the group consisting of: valproic acid, levetiracetam, clobazam, zonisamide, rufinamide, lacosamide, topiramate, clonazepam, perampanel, brivaracetam, gabapentin and phenobarbital.

6. A CBD preparation for use according to any of the preceding claims, wherein the CBD present is isolated from cannabis plant material.

7. A CBD preparation for use according to any of the preceding claims, wherein at least a portion of at least one of the cannabinoids present in the CBD preparation is isolated from cannabis plant material.

8. A CBD preparation for use according to claims 1 to 5, wherein the CBD is present as a synthetic preparation.

9. A CBD preparation for use according to claim 8, wherein at least a portion of at least one of the cannabinoids present in the CBD preparation is prepared synthetically.

10. A CBD preparation for use according to any of the preceding claims, wherein the dose of CBD is greater than 5 mg/kg/day.

11. A CBD preparation for use according to any of the preceding claims, wherein the dose of CBD is 20 mg/kg/day.

12. A CBD preparation for use according to any of claims 1 to 10, wherein the dose of CBD is 25 mg/kg/day.

13. A CBD preparation for use according to any of claims 1 to 10, wherein the dose of CBD is 50 mg/kg/day.

## Patentansprüche

1. Cannabidiol (CBD)-Präparat zur Verwendung bei der Behandlung von Anfällen im Zusammenhang mit 15q11.2-Mikrodeletion, wobei die Anfälle ausgewählt sind aus tonischen, tonisch-klonischen, atonischen, Absence-, fokalen Anfällen ohne Beeinträchtigung und fokalen Anfällen mit sekundärer Generalisierung.

2. CBD-Präparat zur Verwendung nach Anspruch 1, wobei das CBD-Präparat größer als 95 % (w/w) CBD und nicht mehr als 0,15 % (w/w) Tetrahydrocannabinol (THC) umfasst.

3. CBD-Präparat zur Verwendung nach einem der vorhergehenden Ansprüche, wobei das CBD-Präparat größer als oder gleich 98 % (w/w) CBD und weniger als oder gleich 2 % (w/w) andere Cannabinoide umfasst, wobei die weniger als oder gleich 2 % (w/w) andere Cannabinoide die Cannabinoide Tetrahydrocannabinol (THC); Cannabidiol-Cl (CBD-C1); Cannabidivarin (CBDV); und Cannabidiol-C4 (CBD-C4) umfassen und wobei das THC als Gemisch von Trans-THC und cis-THC vorliegt.

4. CBD-Präparat zur Verwendung nach einem der vorhergehenden Ansprüche, wobei das CBD-Präparat in Kombination mit einem oder mehreren begleitenden antiepileptischen Medikamenten (AED) verwendet wird.

5. CBD-Präparat zur Verwendung nach Anspruch 4, wobei das eine oder mehrere AED ausgewählt sind aus der Gruppe bestehend aus: Valproinsäure, Levetiracetam, Clobazam, Zonisamid, Rufinamid, Lacosamid, Topiramat, Clonazepam, Perampanel, Brivaracetam, Gabapentin und Phenobarbital.

6. CBD-Präparat zur Verwendung nach einem der vorhergehenden Ansprüche, wobei das vorliegende CBD aus Cannabispflanzenmaterial isoliert ist.

7. CBD-Präparat zur Verwendung nach einem der vorhergehenden Ansprüche, wobei mindestens ein Teil von mindestens einem der Cannabinoide, die in dem CBD-Präparat vorliegen, aus Cannabispflanzenmaterial isoliert ist.

8. CBD-Präparat zur Verwendung nach Anspruch 1 bis 5, wobei das CBD als synthetisches Präparat vorliegt.

9. CBD-Präparat zur Verwendung nach Anspruch 8, wobei mindestens ein Teil von mindestens einem der in dem CBD-Präparat vorliegenden Cannabinoide synthetisch präpariert ist.

10. CBD-Präparat zur Verwendung nach einem der vorhergehenden Ansprüche, wobei die Dosis an CBD größer als 5 mg/kg/Tag beträgt.

11. CBD-Präparat zur Verwendung nach einem der vorhergehenden Ansprüche, wobei die Dosis an CBD 20 mg/kg/Tag beträgt.

12. CBD-Präparat zur Verwendung nach einem der Ansprüche 1 bis 10, wobei die Dosis an CBD 25 mg/kg/Tag beträgt.

13. CBD-Präparat zur Verwendung nach einem der Ansprüche 1 bis 10, wobei die Dosis an CBD 50 mg/kg/Tag beträgt.

## Revendications

1. Préparation de cannabidiol (CBD) destinée à être utilisée dans le traitement de crises associées à une microdélétion 15q11.2, dans laquelle les crises sont choisies parmi des crises toniques, tonico-cloniques, atoniques, d'absence, des crises focales sans altération et des crises focales avec généralisation secondaire.

2. Préparation de CBD destinée à être utilisée selon la revendication 1, dans laquelle la préparation de CBD comprend plus de 95 % (p/p) de CBD et pas plus de 0,15 % (p/p) de tétrahydrocannabinol (THC).

3. Préparation de CBD destinée à être utilisée selon l'une quelconque des revendications précédentes, dans laquelle la préparation de CBD comprend au moins 98 % (p/p) de CBD et au plus 2 % (p/p) d'autres cannabinoïdes, dans laquelle les autres cannabinoïdes présents à au plus 2 % (p/p) comprennent les cannabinoïdes : tétrahydrocannabinol (THC) ; cannabidiol-C1 (CBD-C1) ; cannabidivarine (CBDV) et cannabidiol-C4 (CBD-C4), et dans laquelle le THC est présent sous forme d'un mélange de trans-THC et de cis-THC.

4. Préparation de CBD destinée à être utilisée selon l'une quelconque des revendications précédentes, dans laquelle la préparation de CBD est utilisée en combinaison avec un ou plusieurs médicaments anti-épileptiques (AED) concomitants.

5. Préparation de CBD destinée à être utilisée selon la revendication 4, dans laquelle le ou les AED sont choisis dans le groupe constitué par : l'acide valproïque, le lévétiracétam, le clobazam, le zonisamide, le rufinamide, le lacosamide, le topiramate, le clonazépam, le pérampanel, le brivaracétam, la gabapentine et le phénobarbital.

6. Préparation de CBD destinée à être utilisée selon l'une quelconque des revendications précédentes, dans laquelle le CBD présent est isolé à partir de matière végétale de cannabis.

7. Préparation de CBD destinée à être utilisée selon l'une quelconque des revendications précédentes, dans laquelle au moins une partie d'au moins l'un des cannabinoïdes présents dans la préparation de CBD est isolée à partir de matière végétale de cannabis.

8. Préparation de CBD destinée à être utilisée selon les revendications 1 à 5, dans laquelle le CBD est présent sous forme d'une préparation synthétique.

9. Préparation de CBD destinée à être utilisée selon la revendication 8, dans laquelle au moins une partie d'au moins l'un des cannabinoïdes présents dans la préparation de CBD est préparée de manière synthétique.

10. Préparation de CBD destinée à être utilisée selon l'une quelconque des revendications précédentes, dans laquelle la dose de CBD est supérieure à 5 mg/kg/jour.

11. Préparation de CBD destinée à être utilisée selon l'une quelconque des revendications précédentes, dans laquelle la dose de CBD est de 20 mg/kg/jour.

12. Préparation de CBD destinée à être utilisée selon l'une quelconque des revendications 1 à 10, dans laquelle la dose de CBD est de 25 mg/kg/jour.

13. Préparation de CBD destinée à être utilisée selon l'une quelconque des revendications 1 à 10, dans laquelle la dose de CBD est de 50 mg/kg/jour.
